# FASCICULE DE BREVET EUROPEEN

(11) **EP 2 032 082 B1**
(45) Date de publication et mention de la délivrance du brevet: **28.03.2012**
(21) Numéro de dépôt: 07788883.2
(22) Date de dépôt: 13.06.2007
(51) Int. Cl.: A61F 2/40, A61B 17/16, A61B 17/17

(54) **PROTHÈSE D'ÉPAULE ET ENSEMBLE D'INSTRUMENTS POUR L'IMPLANTATION DE CETTE PROTHÈSE**
SCHULTERPROTHESE UND INSTRUMENTENSATZ ZUR IMPLANTIERUNG
SHOULDER PROSTHESIS AND SET OF INSTRUMENTS FOR THE IMPLANTATION THEREOF

(30) Priorité: 28.06.2006 FR 0605813
(43) Date de publication de la demande: 11.03.2009
(73) Titulaire: Trois S Ortho, 69009 Lyon (FR); Evolutis, 42720 Briennon (FR); Delince, Philippe, 1000 Bruxelles (BE); Lesur, Etienne, 88060 Epinal Cedex 9 (FR); Oudet, Didier, 37100 Toijrs (FR); Bruchou, François, 78190 Trappes (FR); Chaumeil, Géraud, 81000 Albi (FR)
(72) Inventeur: DELINCE, Philippe, B-1000 Bruxelles (BE); LESUR, Etienne, F-88060 Epinal Cedex 9 (FR); OUDET, Didier, F-37100 Toijrs (FR); BRUCHOU, François, 78190 Trappes (FR); CHAUMEIL, Géraud, 81000 Albi (FR)
(74) Mandataire: Jeannet, Olivier
(86) Numéro de dépôt international: PCT/FR2007/000978
(87) Numéro de publication internationale: WO 2008/000928

(56) Documents cités:
- EP-A- 0 581 667
- WO-A-01/47442
- FR-A1- 2 674 124
- FR-A1- 2 704 747
- US-A1- 5 030 219

## Description

La présente invention concerne une prothèse d'épaule et un ensemble d'instruments pour l'implantation de cette prothèse.

Une prothèse d'épaule comprend classiquement une tige médullaire destinée à être introduite dans le canal médullaire de l'humérus et un élément glénoïdien destiné à être ancré à la glène de l'omoplate, cette tige médullaire et cet élément glénoïdien comportant des surfaces d'articulation permettant leur mobilité. Ces surfaces d'articulation peuvent être constituées par une tête arrondie convexe montée sur la tige et un élément glénoïde fixé à l'omoplate ou, dans les prothèses dites "inversées", par un élément glénoïde monté sur la tige humérale et une tête arrondie convexe fixée à l'omoplate.

Les documents FR 2 704 747, US 5 030 219, FR 2 674 124, WO 01/47442 et EP 0 581 667 illustrent diverses prothèses d'épaules.

Lors de l'implantation d'une prothèse d'épaule, la mise en place de l'élément glénoïdien peut être relativement délicate à réaliser, et la parfaite fixation de cet élément est une condition essentielle du bon fonctionnement, et donc de la pérennité, de la prothèse.

Les prothèses existantes ne donnent pas parfaitement satisfaction de ce point de vue, et un objectif essentiel de la présente invention est de fournir une prothèse d'épaule dont l'élément glénoïdien peut être parfaitement fixé à l'omoplate, et ce de manière relativement facile et rapide à mettre en oeuvre.

Selon l'invention,
- l'élément glénoïdien comprend une queue distale d'ancrage et une collerette proximale, la queue distale d'ancrage ayant une section transversale substantiellement en forme de " I ", c'est-à-dire avec une âme centrale et deux parois latérales sensiblement perpendiculaires à cette âme centrale, et ayant une forme vrillée hélicoïdalement, et la collerette proximale étant percée d'au moins un trou ; et
- la prothèse comprend au moins une vis destinée à être engagée dans le trou de ladite collerette et à être insérée dans l'os.

L'élément glénoïdien est ainsi mis en place dans l'omoplate par impaction hélicoïdale dans un perçage de section correspondante préalablement aménagé dans l'omoplate au moyen d'un instrument approprié, jusqu'à venue de la collerette au ,contact de l'os, puis au moins une vis est mise en place au travers de la collerette puis dans l'os, afin de bloquer le pivotement de cet élément glénoïdien.

Cette mise en place par impaction hélicoïdale, combinée à la section en "I" de la queue distale d'ancrage, permet une parfaite fixation de l'élément glénoïdien, avec une prise d'appui dans l'os de surface importante.

L'ensemble d'instruments comprend, selon l'invention, un instrument de perçage, muni d'une partie de perçage et de creusement de l'os de la glène de l'omoplate ayant une même forme et des mêmes dimensions que celles de la queue distale d'ancrage de l'élément glénoïdien, ou des dimensions légèrement inférieures.

La queue distale d'ancrage hélicoïdale peut comprendre un revêtement ostéo-inducteur, notamment un revêtement poreux d'hydroxy-apatite de calcium.

Avantageusement, l'extrémité distale de la queue distale d'ancrage est aménagée à la manière de l'extrémité de coupe d'un foret, c'est-à-dire présente des faces distales inclinées du côté proximal depuis l'axe de la queue distale d'ancrage et biseautées selon des angles de dépouille inverses.

La queue distale d'ancrage peut ainsi être plus ou moins autoforante ; elle peut dès lors être introduite dans un perçage osseux de dimensions ajustées, ou légèrement inférieures, aux siennes, ce qui assure sa parfaite immobilisation par rapport à l'os.

Selon une possibilité, la queue distale d'ancrage comprend un alésage axial permettant son engagement sur une broche de guidage faisant partie de l'ensemble d'instruments selon l'invention ; l'instrument de perçage comprend dans ce cas également un alésage axial permettant son engagement sur cette broche.

Cet instrument de perçage peut ainsi être précisément guidé par cette broche lors du perçage de l'os puis l'élément glénoïdien peut à son tour être précisément guidé par cette même broche lors de sa mise en place.

La collerette proximale présente avantageusement une face distale de forme arrondie convexe. Cette face permet à la collerette de venir au contact de l'os selon une surface importante.

Cette face peut également comprendre un revêtement ostéo-inducteur.

Au moins un des trous de la collerette peut être délimité par une paroi conique ou en portion de sphère creuse, permettant d'implanter la vis selon une pluralité de positions angulaires possibles par rapport à l'élément glénoïdien.

Une latitude dans le positionnement de la vis est ainsi créée, permettant l'implantation de cette vis dans les meilleures conditions.

La collerette comprend de préférence plusieurs trous, notamment quatre trous, pour permettre un choix de positionnement d'une ou plusieurs vis.

L'élément glénoïdien comprend également des moyens de montage sur lui d'un élément formant la surface d'articulation. Ces moyens de montage peuvent notamment comprendre un plot tronconique à faible pente, sur lequel peut être engagé un élément formant la surface d'articulation, cet élément comprenant une cavité tronconique à faible pente correspondante.

Ce plot peut comprendre un alésage taraudé axial permettant d'assurer le montage de l'élément formant la surface d'articulation au moyen d'une vis. Une telle vis est notamment utile lorsque l'élément formant la surface d'articulation comprend une tête en céramique. Le trou de cette vis peut éventuellement servir à l'ablation de la surface d'articulation.

L'ensemble d'instruments selon l'invention peut également comprendre un instrument de repérage de la portion osseuse à percer, présentant une tête circulaire aplatie dans laquelle est aménagée une ouverture de même profil que la section transversale en "I" de la queue médullaire d'ancrage.

L'invention sera bien comprise, et d'autres caractéristiques et avantages de celle-ci apparaîtront, en référence au dessin schématique annexé, ce dessin représentant, à titre d'exemple non limitatif, une forme de réalisation préférée de la prothèse d'épaule qu'elle concerne.
La figure 1 est une vue de côté de différents éléments constituant cette prothèse, avant assemblage ;
la figure 2 est une vue en perspective de la tige humérale que comprend cette prothèse ;

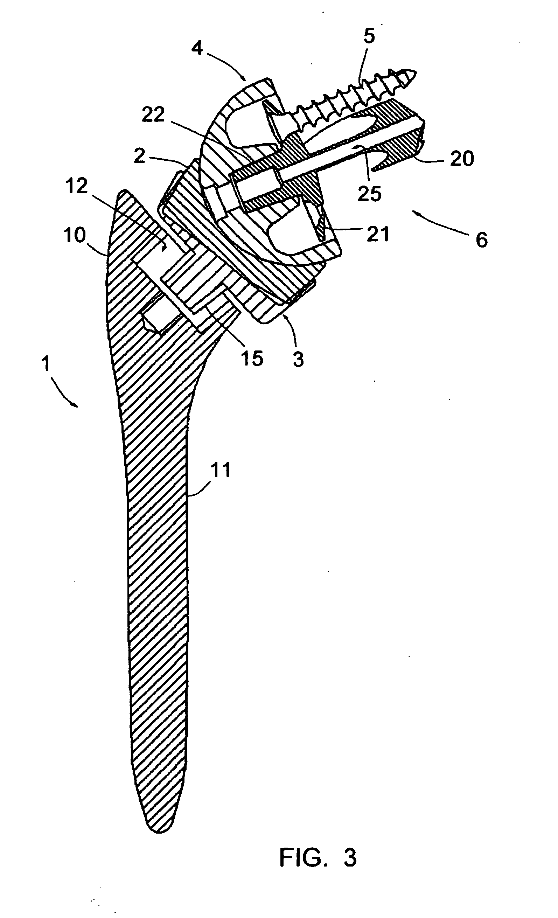

la figure 3 est une vue en coupe axiale de différents éléments constituant cette prothèse, après assemblage ;
la figure 4 est une vue en perspective, par le côté distal, d'un élément glénoïdien que comprend cette prothèse ;
la figure 5 est une vue en perspective, par le côté proximal, de cet élément glénoïdien ;
la figure 6 est une vue de cet élément glénoïdien de côté, et
la figure 7 est une vue de cet élément glénoïdien en bout, par le côté distal.

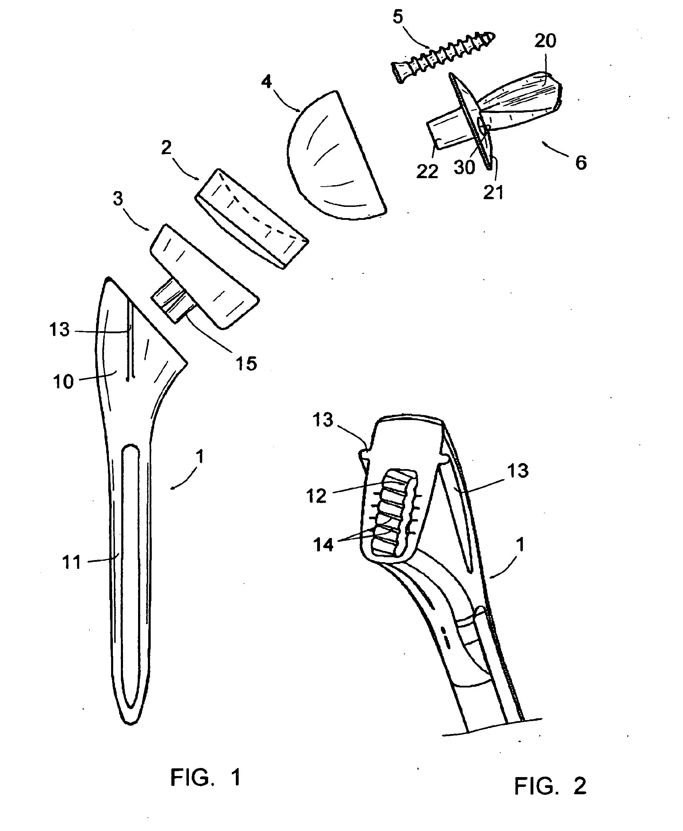

La figure 1 représente différents éléments permettant de constituer une prothèse d'épaule, à savoir une tige médullaire humérale 1, un élément 2 formant une surface d'articulation glénoïde, une embase 3 de montage de cet élément 2 sur la tige humérale 1, une sphère glénoïdienne 4 formant une surface d'articulation arrondie convexe propre à coopérer avec la surface d'articulation de l'élément 2, une vis 5 d'ancrage osseux et un élément glénoïdien 6.

La tige humérale 1 présente une partie métaphysaire élargie 10 et une partie épiphysaire effilée 11. La partie métaphysaire 10 présente une cavité 12 allongée dans le plan interne-externe, débouchant dans une face plane qui limite cette partie métaphysaire 10 du côté proximal, et comprend des nervures 13 sur ses faces antérieures et postérieures, assurant sa stabilisation par rapport à l'os. La tige humérale 1 peut éventuellement recevoir un revêtement biologique.

La cavité 12 comprend une pluralité de saillies 14 aménagées au niveau des parois antérieure et postérieure qui la délimitent longitudinalement ; ladite embase 3 comprend un plot 15 destiné à être engagé dans cette cavité 12, dont la largeur est nettement inférieure à la longueur de la cavité 12 et dont les faces latérales présentent une forme complémentaire de celle des saillies 14. Cette cavité 12 et ce plot 15 forment ainsi un système d'indexation permettant une pluralité de positions possibles de l'embase 3 par rapport à la tige 1, et donc un réglage de la position de l'élément 2 par rapport à cette tige 1.

L'élément 2 est en matériau favorisant le glissement, notamment en polyéthylène à haute densité ou en céramique.

L'embase 3 comprend, outre le plot précité, un logement de réception ajustée de l'élément 2.

La sphère glénoïdienne 4 présente une forme arrondie, hémisphérique ou en calotte sphérique. Elle peut être métallique ou peut comprendre un élément en céramique, formant ladite surface d'articulation, cet élément étant monté sur un insert en matériau métallique.

La vis 5 est une vis à os de type classique.

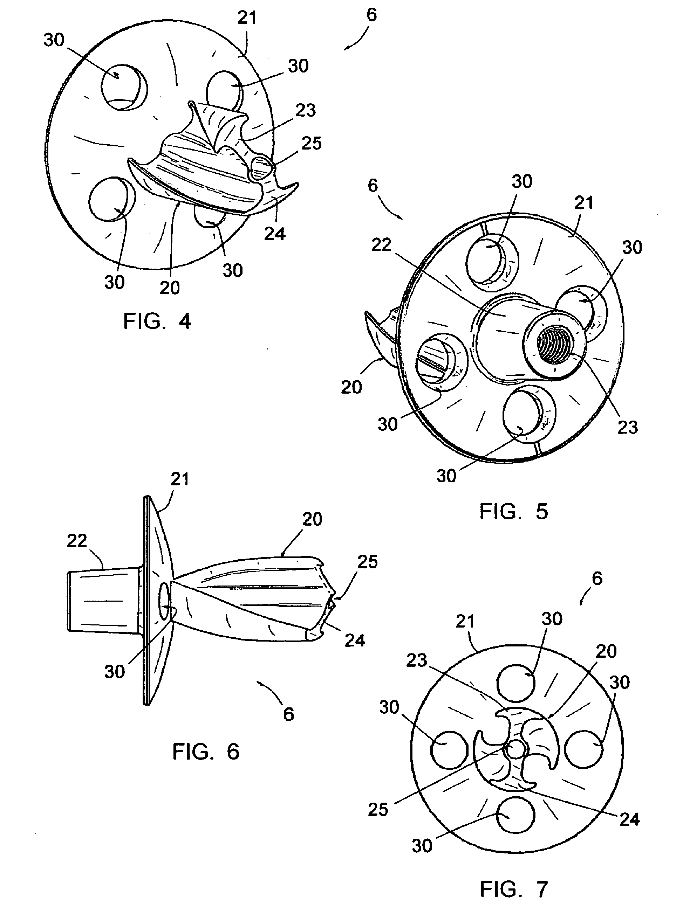

L'élément glénoïdien 6 est plus particulièrement visible sur les figures 4 à 7. Ainsi que cela apparaît en référence à ces figures, cet élément 6 comprend une queue distale d'ancrage 20, une collerette proximale 21 et un plot proximal 22 de montage de la sphère glénoïdienne 4.

La queue distale d'ancrage 20 a une section transversale substantiellement en forme de " I ", c'est-à-dire avec une âme centrale et deux parois latérales sensiblement perpendiculaires à cette âme centrale, et a une forme vrillée hélicoïdalement. Son extrémité distale est aménagée à la manière de l'extrémité de coupe d'un foret, c'est-à-dire présente des faces distales 23, 24 inclinées du côté proximal depuis l'axe de la queue distale d'ancrage 20 (cf. figure 6) et biseautées selon des angles de dépouille inverses (cf. figure 4).

La queue distale d'ancrage 20 comprend un alésage axial 25 permettant son engagement sur une broche de guidage (non représentée).

En outre, cette queue distale d'ancrage 20 comprend un revêtement ostéo-inducteur, notamment un revêtement poreux d'hydroxy-apatite de calcium.

La collerette proximale 21 présente une face distale de forme arrondie convexe, comprenant également un revêtement ostéo-inducteur. Elle est percée de quatre trous 30 régulièrement répartis sur sa périphérie, qui sont chacun délimités par une paroi en portion de sphère creuse, permettant d'implanter la vis 5, destinée à être engagée au travers de l'un de ces trous 30, selon une pluralité de positions angulaires possibles par rapport à l'élément glénoïdien 6.

Le plot 22 présente une forme tronconique à faible pente et peut recevoir la sphère glénoïdienne 4 emmanchée sur lui, cette tête comprenant une cavité tronconique à faible pente correspondante.

Le plot 22 comprend en outre un alésage taraudé axial 23 permettant d'assurer le montage de la sphère glénoïdienne 4 au moyen d'une vis, si nécessaire et permettant éventuellement l'extraction de la sphère glénoïdienne par système de type arrache moyeu.

L'élément glénoïdien 6 est destiné à être implanté au moyen d'un ensemble d'instruments (non représentés) incluant un instrument de repérage et un instrument de perçage.

L'instrument de repérage permet de repérer la portion osseuse à percer ; il présente une tête circulaire aplatie dans laquelle est aménagée une ouverture de même profil que la section transversale en " I " de la queue médullaire d'ancrage 20.

L'instrument de perçage est muni d'une partie de perçage et de creusement de l'os de la glène de l'omoplate, ayant une même forme et des mêmes dimensions que celles de la queue distale d'ancrage 20, ou des dimensions légèrement inférieures. L'instrument de perçage comprend également un alésage axial permettant son engagement sur la même broche de guidage que celle sur laquelle peut être engagé l'élément glénoïdien 6.

En pratique, la zone à percer est repérée au moyen de l'instrument de repérage puis la broche de guidage est mise en place. L'instrument de perçage est alors utilisé pour percer et creuser la glène de l'omoplate selon la forme de la queue distale d'ancrage de l'élément. Cet instrument est retiré après perçage puis l'élément glénoïdien 6 est mis en place par impaction hélicoïdale jusqu'à venue de la collerette 21 au contact de l'os. Une ou plusieurs vis 5 est alors mise en place au travers de l'un des trous 30. La sphère glénoïdienne 4 est ensuite engagée sur le plot 22 et, le cas échéant, assurée dans sa position de montage au moyen d'une vis mise en place dans l'alésage 23.

Parallèlement, la tige 1 est mise en place dans l'humérus, et l'ensemble élément 2 - embase 3 est mis en place sur elle, dans la position adéquate, par engagement du plot 15 dans la cavité 12.

Comme cela apparaît de ce qui précède, l'invention fournit une prothèse d'épaule dont l'élément glénoïdien présente les avantages déterminants de pouvoir être parfaitement fixé à l'omoplate, et ce de manière relativement facile et rapide à mettre en oeuvre.

Il va de soi que l'invention n'est pas limitée à la forme de réalisation décrite ci-dessus à titre d'exemple mais qu'elle s'étend à toutes les formes de réalisations couvertes par les revendications ci-annexées.

## Revendications

1. Prothèse d'épaule, comprenant un élément glénoïdien (6) destiné à être ancré à la glène de l'omoplate,
- cet élément glénoïdien (6) comprenant une queue distale d'ancrage (20) et une collerette proximale (21), étant percée d'au moins un trou (30) ; et
- la prothèse comprenant au moins une vis (5) destinée à être engagée dans le trou (30) de ladite collerette (21) et à être insérée dans l'os*,* **caractérisée en ce que** la queue distale d'ancrage (20) a une section transversale substantiellement en forme de "I", c'est-à-dire avec une âme centrale et deux parois latérales sensiblement perpendiculaires à cette âme centrale, et a une forme vrillée hélicoïdalement.

2. Prothèse d'épaule selon la revendication 1, **caractérisée en ce que** la queue distale d'ancrage hélicoïdale (20) comprend un revêtement ostéo-inducteur, notamment un revêtement poreux d'hydroxy-apatite de calcium.

3. Prothèse d'épaule selon la revendication 1 ou la revendication 2, **caractérisée en ce que** l'extrémité distale de la queue distale d'ancrage (20) est aménagée à la manière de l'extrémité de coupe d'un foret, c'est-à-dire présente des faces distales (23, 24) inclinées du côté proximal depuis l'axe de la queue distale d'ancrage (20) et biseautées selon des angles de dépouille inverses.

4. Prothèse d'épaule selon l'une des revendications 1 à 3, **caractérisée en ce que** la queue distale d'ancrage (20) comprend un alésage axial (25) permettant son engagement sur une broche de guidage.

5. Prothèse d'épaule selon l'une des revendications 1 à 4, **caractérisée en ce que** la collerette proximale (21) présente une face distale de forme arrondie convexe.

6. Prothèse d'épaule selon l'une des revendications 1 à 5, **caractérisée en ce que** la face distale de la collerette proximale (21) comprend un revêtement ostéo-inducteur.

7. Prothèse d'épaule selon l'une des revendications 1 à 6, **caractérisée en ce qu'**au moins un des trous (30) de la collerette (21) est délimité par une paroi conique ou en portion de sphère creuse, permettant d'implanter la vis (5) selon une pluralité de positions angulaires possibles par rapport à l'élément glénoïdien (6).

8. Prothèse d'épaule selon l'une des revendications 1 à 7, **caractérisée en ce que** la collerette (21) comprend plusieurs trous (30), notamment quatre trous.

9. Prothèse d'épaule selon l'une des revendications 1 à 8, **caractérisée en ce que** l'élément glénoïdien (6) comprend des moyens de montage sur lui d'un élément formant la surface d'articulation, incluant notamment un plot (22) tronconique à faible pente sur lequel peut être engagé un élément (4) formant la surface d'articulation, cet élément (4) comprenant une cavité tronconique à faible pente correspondante.

10. Prothèse d'épaule selon la revendication 9, **caractérisée en ce que** le plot (22) comprend un alésage taraudé axial (23) permettant d'assurer le montage de l'élément (4) formant la surface d'articulation au moyen d'une vis.

11. Ensemble d'instruments pour l'implantation de la prothèse selon l'une des revendications 1 à 10, **caractérisé en ce qu'**il comprend un instrument de perçage, muni d'une partie de perçage et de creusement de l'os de la glène de l'omoplate ayant une même forme et des mêmes dimensions que celles de la queue distale d'ancrage (20) de l'élément glénoïdien (6), ou des dimensions légèrement inférieures.

12. Ensemble d'instruments selon la revendication 11, **caractérisé en ce que** l'instrument de perçage comprend un alésage axial permettant son engagement sur une broche de guidage.

13. Ensemble d'instruments selon la revendication 11 ou la revendication 12, **caractérisé en ce qu'**il comprend un instrument de repérage de la portion osseuse à percer, présentant une tête circulaire aplatie dans laquelle est aménagée une ouverture de même profil que la section transversale en "I" de la queue médullaire d'ancrage.

## Claims

1. Shoulder prosthesis, comprising a glenoid element (6) intended to be attached to the scapula;
- this glenoid element (6) comprising a distal anchoring tail (20) and a proximal flange (21) being perforated with at least one hole (30); and
- the prosthesis comprising at least one screw (5) intended to be engaged into the hole (30) of said flange (21) and to be inserted into the bone, **characterized in that** the distal anchoring tail (20) has a substantially "I-shaped" cross-section, i.e. with a central core and two side walls substantially perpendicular to this central core, and has a helicoidally twisted shape.

2. Shoulder prosthesis according to claim 1, **characterized in that** the distal helicoidal anchoring tail (20) comprises an osteo-inductive coating, notably a porous coating of calcium hydroxyapatite.

3. Shoulder prosthesis according to claim 1 or claim 2, **characterized in that** the distal end of the distal anchoring tail (20) is arranged in the same way as the cutting end of a drilling bit, i.e. it has distal faces (23, 24) tilted on the proximal side from the axis of the distal anchoring tail (20) and beveled according to inverse relief angles.

4. Shoulder prosthesis according to claim 1 to 3, **characterized in that** the distal anchoring tail (20) comprises an axial bore (25) allowing it to be engaged onto a guiding pin.

5. Shoulder prosthesis of claim 1 to 4, **characterized in that** the proximal flange (21) has a distal face with a convex rounded shape.

6. Shoulder prosthesis of claim 1 to 5, **characterized in that** the proximal flange (21) comprises an osteo-inductive coating.

7. Shoulder prosthesis of claim 1 to 6, **characterized in that** at least one of the holes (30) of the flange (21) may be delimited by a conical wall or a hollow sphere portion, allowing the screw (5) to be implanted according to a plurality of possible angular positions relatively to the glenoid element (6).

8. Shoulder prosthesis of claim 1 to 7, **characterized in that** the flange (21) comprises several holes (30) notably four holes.

9. Shoulder prosthesis of claim 1 to 8, **characterized in that** the glenoid element (6) comprises mounting means on it for an element forming the jointed surface, including notably a low-pitched frustro-conical stud (22), onto which an element (4) forming the jointed surface may be engaged, this element (4) comprising a corresponding low-pitched frustro-conical cavity.

10. Shoulder prosthesis of claim 9, **characterized in that** the stud (22) comprises an axial tapped bore (23) with which the mounting of the element (4) forming the jointed surface may be ensured by means of a screw.

11. A set of instruments for implanting the prosthesis of anyone of claims 1 to 10, **characterized in that** it comprises a perforating instrument, provided with a portion for perforating and hollowing out the bone of the socket of the scapula having a same shape and same dimensions as those of the distal anchoring tail (20) of the glenoid element (6), or slightly smaller dimensions.

12. The set of instruments of claim 11, **characterized in that** the perforating instrument comprises an axial bore allowing it to be engaged onto a guiding pin.

13. The set of instruments of claim 11 or claim 12, **characterized in that** it comprises an instrument for locating the bone portion to be perforated, having a flattened circular head in which an aperture is made of the same profile as that of the "I-shaped" cross-section of the medullary anchoring tail.

## Patentansprüche

1. Schulterprothese, umfassend ein Gelenkpfannenelement (6), das dazu bestimmt ist, an der Gelenkpfanne des Schulterblatts verankert zu werden,
- wobei dieses Gelenkpfannenelement (6) einen distalen Verankerungsschaft (20) und einen proximalen Kragen (21) umfasst, durch den mindestens ein Loch (30) gebohrt ist; und
- wobei die Prothese mindestens eine Schraube (5) umfasst, die dazu bestimmt ist, in das Loch (30) des Kragens (21) eingefügt und in den Knochen eingeführt zu werden, **dadurch gekennzeichnet, dass** der distale Verankerungsschaft (20) einen im Wesentlichen "I"-förmigen Querschnitt, das heißt, mit einem Mittelsteg und zwei seitlichen Wänden, die im Wesentlichen senkrecht zu diesem Mittelsteg verlaufen, und eine schraubenförmig verdrehte Form aufweist.

2. Schulterprothese nach Anspruch 1, **dadurch gekennzeichnet, dass** der distale, schraubenförmige Verankerungsschaft (20) eine osteoinduktive Beschichtung, insbesondere eine poröse Beschichtung aus Calcium-Hydroxylapatit, aufweist.

3. Schulterprothese nach Anspruch 1 oder 2, **dadurch gekennzeichnet, dass** das distale Ende des distalen Verankerungsschafts (20) wie das Schneidende eines Bohrers ausgeführt ist, das heißt, es weist distale Flächen (23, 24) auf, die ausgehend von der Achse des distalen Verankerungsschafts (20) proximalseitig geneigt und gemäß umgekehrten Freiwinkeln abgeschrägt sind.

4. Schulterprothese nach einem der Ansprüche 1 bis 3, **dadurch gekennzeichnet, dass** der distale Verankerungsschaft (20) eine axiale Bohrung (25) umfasst, die sein Aufsetzen auf einen Führungsstift ermöglicht.

5. Schulterprothese nach einem der Ansprüche 1 bis 4, **dadurch gekennzeichnet, dass** der proximale Kragen (21) eine distale Fläche mit abgerundeter, konvexer Form aufweist.

6. Schulterprothese nach einem der Ansprüche 1 bis 5, **dadurch gekennzeichnet, dass** die distale Fläche des proximalen Kragens (21) eine osteoinduktive Beschichtung aufweist.

7. Schulterprothese nach einem der Ansprüche 1 bis 6, **dadurch gekennzeichnet, dass** mindestens eines der Löcher (30) des Kragens (21) von einer Wand begrenzt ist, die konisch oder als hohler Kugelabschnitt ausgeführt ist, wodurch das Einsetzen der Schraube (5) gemäß mehreren möglichen Winkelpositionen in Bezug auf das Gelenkpfannenelement (6) ermöglicht wird.

8. Schulterprothese nach einem der Ansprüche 1 bis 7, **dadurch gekennzeichnet, dass** der Kragen (21) mehrere Löcher (30), insbesondere vier Löcher, umfasst.

9. Schulterprothese nach einem der Ansprüche 1 bis 8, **dadurch gekennzeichnet, dass** das Gelenkpfannenelement (6) Mittel umfasst, um auf ihm ein Element zu befestigen, das die Gelenksoberfläche bildet, insbesondere umfassend ein kegelstumpfförmiges Verbindungsstück (22) mit schwacher Neigung, auf das ein Element (4) aufgesetzt werden kann, das die Gelenksoberfläche bildet, wobei dieses Element (4) eine kegelstumpfförmige Aushöhlung mit entsprechender schwacher Neigung umfasst.

10. Schulterprothese nach Anspruch 9, **dadurch gekennzeichnet, dass** das Verbindungsstück (22) eine axiale Gewindebohrung (23) umfasst, die es ermöglicht, die Befestigung des Elements (4), das die Gelenksoberfläche bildet, mittels einer Schraube sicherzustellen.

11. Instrumentensatz zur Implantierung der Prothese nach einem der Ansprüche 1 bis 10, **dadurch gekennzeichnet, dass** er ein Bohrinstrument umfasst, das mit einem Teil zum Anbohren und Aushöhlen des Knochens der Gelenkpfanne des Schulterblatts ausgestattet ist, der die gleiche Form und die gleichen Abmessungen wie jene des distalen Verankerungsschafts (20) des Gelenkpfannenelements (6) oder leicht kleinere Abmessungen aufweist.

12. Instrumentensatz nach Anspruch 11, **dadurch gekennzeichnet, dass** das Bohrinstrument eine axiale Bohrung umfasst, die sein Aufsetzen auf einen Führungsstift ermöglicht.

13. Instrumentensatz nach Anspruch 11 oder 12, **dadurch gekennzeichnet, dass** er ein Instrument zur Kennzeichnung des anzubohrenden Knochenabschnitts umfasst, das einen kreisförmigen, abgeflachten Kopf aufweist, in dem eine Öffnung mit dem gleichen Profil wie der "I"-förmige Querschnitt des medullären Verankerungsschafts ausgebildet ist.
